(19) Europäisches Patentamt
European Patent Office
Office européen des brevets

(11) Veröffentlichungsnummer: **0 504 817 A1**

(12) # EUROPÄISCHE PATENTANMELDUNG

(21) Anmeldenummer: **92104612.4**

(22) Anmeldetag: **17.03.92**

(51) Int. Cl.5: **C07D 239/52**, A01N 47/36, C07C 311/48

(30) Priorität: **21.03.91 CH 871/91**

(43) Veröffentlichungstag der Anmeldung:
**23.09.92 Patentblatt 92/39**

(84) Benannte Vertragsstaaten:
**CH DE FR GB LI**

(71) Anmelder: **LONZA AG**
**Gampel/Wallis Geschäftsleitung Basel**
**CH-4002 Basel(CH)**

(72) Erfinder: **Griffiths, Gareth, Dr.**
**Bäretstrasse 6**
**Visp (Kanton Wallis)(CH)**
Erfinder: **Warm, Aleksander, Dr.**
**Bifigastrasse**
**Visperterminen (Kanton Wallis)(CH)**
Erfinder: **Previdoli, Felix, Dr.**
**Bielastrasse 49**
**Brig (Kanton Wallis)(CH)**
Erfinder: **Ryan, Gary, Dr.**
**22 Brookfield Av. Thornton near Blackpool**
**Lancashire FY5 4DS(GB)**

(74) Vertreter: **Weinhold, Peter, Dr. et al**
**Patentanwälte Dipl.-Ing. G. Dannenberg Dr.**
**P. Weinhold Dr. D. Gudel Dipl.-Ing. S.**
**Schubert Dr. P. Barz Siegfriedstrasse 8**
**W-8000 München 40(DE)**

(54) **Verfahren zur Herstellung von 1-(N-Metylsulfonyl-N-methylaminosulfonyl)-3-(2-pyrimidyl)-harnstoffderivaten.**

(57) Beschrieben wird ein neues Verfahren zur Herstellung von 1-(N-Methylsulfonyl-N-methylaminosulfonyl)-3-(2-pyrimidyl)-harnstoffderivate. Dabei wird zunächst Methansulfonsäure-N-methylamid mit Chlorsulfonylisocyanat in 1-Methylsulfonyl-1-methyl-3-(N-methylsulfonyl-N-methylaminosulfonyl)-harnstoff überführt und anschliessend mit einem 2-Aminopyrimidin in das Endprodukt überführt.

EP 0 504 817 A1

Die Erfindung betrifft ein neues Verfahren zur Herstellung von 1-(N-Methansulfonyl-N-methylaminosulfonyl)-3-(2-pyrimidyl)-harnstoffderivaten sowie ein neues Zwischenprodukt zu deren Herstellung.

Die 1-(N-Methansulfonyl-N-methylaminosulfonyl)-3-(2-pyrimidyl)-harnstoffderivate, die im folgenden mit MMSPH abgekürzt werden, finden beispielsweise bei der Wachstumsregulierung von Pflanzen Anwendung (EP-PS 0 131 258).

Ein Verfahren zur Herstellung von MMSPH ist bereits in der EP-PS 0 131 258 beschrieben. Dabei wird zunächst Methansulfonyl-N-methylamino-sulfonylisocyanat (MSMASI), ausgehend von Methansulfonylmethylamin (MMSA) und Chlorsulfonylisocyanat (CSI), hergestellt. Anschliessend wird MSMASI mit 2-Amino-4,6-dimethoxypyrimidin (ADMP) in das entsprechende MMSPH überführt.
Ein grosser Nachteil dieses Verfahrens liegt darin, dass es, bedingt durch die Neigung zur autokatalytischen Zersetzung von MSMASI, aus sicherheitstechnischen Gründen nur begrenzt anwendbar ist, zumal bei relativ hohen Temperaturen gearbeitet werden muss.

Aufgabe der vorliegenden Erfindung war es, diesen Nachteil auszuschalten und ein sicherheitstechnisch besseres Verfahren zur Verfügung zu stellen.
Diese Aufgabe wurde gemäss Patentanspruch 1 gelöst.

Erfindungsgemäss wird das Verfahren zur Herstellung von MMSPH, worin $R_1$, $R_2$ und $R_3$ gleich oder verschieden sind und ein Wasserstoffatom, eine $C_1$-$C_4$-Alkoxygruppe, eine Hydroxylgruppe oder ein Halogenatom bedeuten, derart durchgeführt, dass man in der ersten Stufe MMSA der Formel

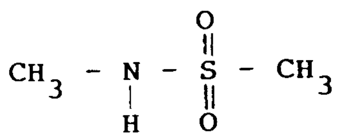

II

mit CSI in das 1-Methansulfonyl-1-methyl-3-(N-methansulfonyl-N-methylaminosulfonyl)-harnstoff-derivat (MMMSH) der Formel

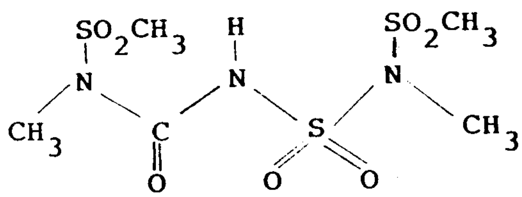

III

überführt, dieses gegebenenfalls isoliert und dieses dann in der zweiten Stufe mit einem 2-Aminopyrimidin der Formel

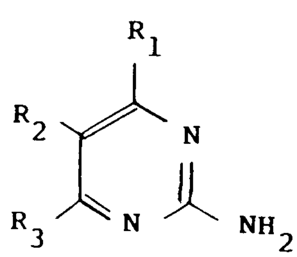

IV

worin $R_1$, $R_2$ und $R_3$ die genannte Bedeutung haben, in das Endprodukt überführt.

Zweckmässig wird die Umsetzung in der ersten Stufe mit 2 mol MMSA, bezogen auf 1 mol CSI, durchgeführt.

Zweckmässig wird die Umsetzung in der ersten Stufe bei einer Temperatur von 50 bis 100 °C durchgeführt, vorzugsweise bei einer Temperatur von 50 bis 85 °C.

Die erste Stufe wird zweckmässig in einem inerten Lösungsmittel durchgeführt.
Als inerte Lösungsmittel können Aromaten, aliphatische oder aromatische Halogenkohlenwasserstoffe, Ether oder niedrigsiedende Alkane angewendet werden. Als Vertreter dieser Lösungsmittel können beispielsweise Chlorbenzol, Hexan, Toluol oder Dichlorethan angewendet werden.

Vorzugsweise wird als inertes Lösungsmittel Dichlorethan verwendet.

Das so gewonnene MMMSH der Formel

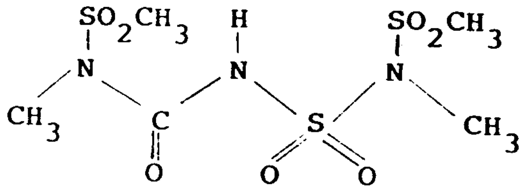

III

ist Bestandteil der Erfindung und wird durch Umsetzung mit einem 2-Aminopyrimidin in MMSPH überführt. Als 2-Aminopyrimidin wird zweckmässig ADMP eingesetzt.

Zweckmässig wird in der zweiten Stufe das ADMP mit dem MMMSH aequimolar eingesetzt.

Die Umsetzung in der zweiten Stufe wird zweckmässig bei einer Temperatur von 50 bis 100°C, vorzugsweise von 50 bis 85°C, durchgeführt.

Zweckmässig wird die zweite wie die erste Stufe in einem inerten Lösungsmittel durchgeführt. Als inertes Lösungsmittel können die gleichen wie die zuvor beschriebenen angewendet werden.

Nach einer üblichen Umsetzungsdauer von 3 bis 24 h kann das MMSPH durch Ausfällen erhalten werden.

Das Verfahren zur Herstellung von MMSPH kann mit oder ohne Isolation von MMMSH durchgeführt werden.

In einer bevorzugten Ausführungsform wird das Verfahren als Eintopfvariante ohne Isolation des Zwischenproduktes durchgeführt, wobei das in der zweiten Stufe anfallende MMSA recyclisiert wird.

Beispiel

a) Herstellung von MMMSH

Eine Lösung von MMSA (20 g, 183,3 mmol) in Chlorbenzol (60 ml) wurde auf 80°C erhitzt. Bei dieser Temperatur wurde innerhalb 15 min CSI (13 g, 8 ml, 91,1 mmol) hinzugetropft. Nach 2 h Nachrühren bei 80°C wurde ein Niederschlag erhalten, der dann abgesaugt wurde. Diese Kristalle wurden dann mit n-Hexan gewaschen und bei 20°C im Vakuum (50 mbar) getrocknet.

Man erhielt 26,9 g Produkt als weisse Kristalle, entsprechend einer Ausbeute von 90,5%, bezogen auf eingesetztes CSI.

Schmp.: 126-128°C

| CHN-Analyse: | | | |
|---|---|---|---|
| ber. | C 18,57% | H 4,00% | N 12,989% |
| gef. | C 18,4 | H 3,8 | N 13,1 |

$^1$H-NMR ($CDCl_3$, 300 MHz) δ in ppm: 10,12 (br. s, 1H)
3,52 (s, 3H)
3,31 (s, 6H)
3,24 (s, 3H)

b) Herstellung von MMSPH

Zu einer Lösung von ADMP (2,73 g, 12,7 mmol) in Chlorbenzol (20 ml) bei 50°C wurde MMMSH (4,52 g, 12,7 mmol) als Feststoff hinzugegeben. Das Reaktionsgemisch wurde noch 5 h bei 55°C nachgerührt und auf Raumtemperatur abgekühlt, wobei das Produkt ausfiel. Dieser Niederschlag wurde abgesaugt und bei 50°C im Vakuum (50 mbar) getrocknet.

Es wurden 4,47 g MMSPH erhalten, mit einer Reinheit von 90,7% gemäss HPLC, entsprechend einer Ausbeute von 86,4%, bezogen auf eingesetztes ADMP.

c) Herstellung von MMSPH als Eintopfvariante

Eine Lösung von MMSA (99,48%ig) (21,94g, 0,2 mol) in Dichlorethan (100 ml) wurde auf 80 °C erhitzt. Bei dieser Temperatur wurde innerhalb 15 min CSI (11,32 g, 0,08 mol) hinzugetropft. Das Reaktionsgemisch wurde dann 2 h lang bei 80 °C gerührt. Die Reaktionsgemischung wurde dann auf 50 °C gekühlt.
Anschliessend wurde zu dieser gelben Lösung eine Lösung von ADMP (99,2%ig) (15,02 g, 0,1 mol) in Dichlorethan (20 ml) zugegeben, wobei sich ein Niederschlag bildete. Die Reaktionsmischung wurde dann 2 h lang bei 50 °C gerührt.
Die Suspension wurde gekühlt und der Niederschlag abfiltriert. Der Feststoff wurde mit Methanol (3 X 30 ml) gewaschen. Das Produkt wurde bei 35 °C im Vakuum (25 mbar) getrocknet. Man erhielt 26,95 g Produkt in Form von weissen Kristallen, mit einer Reinheit von 100% (gemäss HPLC), entsprechend einer Ausbeute von 91%, bezogen auf eingesetztes CSI.
Schmp: 160,5-162,5 °C

| CHN-Analyse: | | | |
|---|---|---|---|
| ber.: | C 29,3%, | H 4,1%, | N 19,0% |
| gef.: | C 29,1%, | H 4,1%, | N 18,8% |

| | |
|---|---|
| IR (0,5% in KBr): | 1711, 1616, 1572, 1512 $cm^{-1}$ |
| $^{1}$H-NMR (DMSO, 300 MHz) δ in ppm: | 13,1 (br.s, 1H) |
| | 10,9 (s, 1H) |
| | 6,02 (s, 1H) |
| | 3,9 (s, 6H) |
| | 3,43 (s, 3H) |
| | 3,38 (s, 3H) |

**Patentansprüche**

**1.** Verfahren zur Herstellung von 1-(N-Methansulfonyl-N-methylaminosulfonyl)-3-(2-pyrimidyl)-harnstoffderivaten der Formel

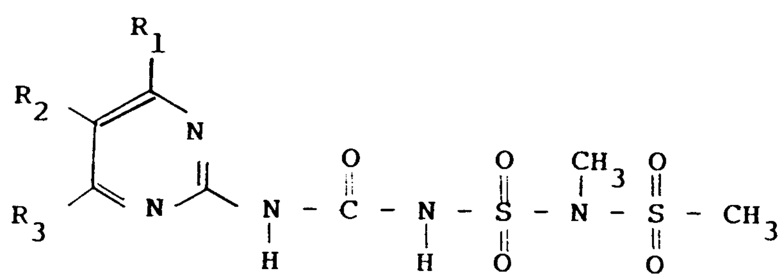

I

worin $R_1$, $R_2$ und $R_3$ gleich oder verschieden sind und ein Wasserstoffatom, eine $C_1$-$C_4$-Alkoxygruppe, eine Hydroxylgruppe oder ein Halogenatom bedeuten, dadurch gekennzeichnet, dass man in der ersten Stufe Methansulfonylmethylamin der Formel

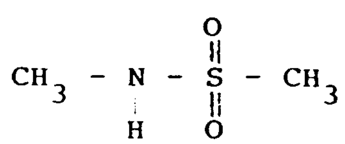

II

mit Chlorsulfonylisocyanat in das 1-Methansulfonyl-1-methyl-3-(N-methansulfonyl-N-methylaminosulfonyl)-harnstoff-derivat der Formel

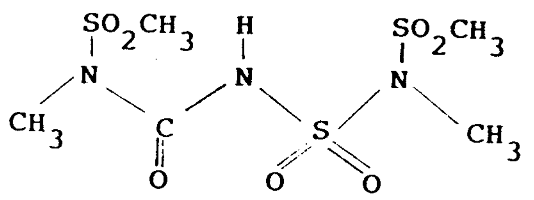

III

überführt, dieses gegebenenfalls isoliert und dieses dann in der zweiten Stufe mit einem 2-Aminopyrimidin der Formel

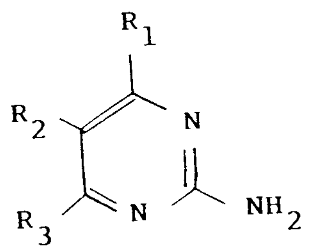

IV

worin $R_1$, $R_2$ und $R_3$ die genannte Bedeutung haben, in das Endprodukt überführt.

2. Verfahren nach Patentanspruch 1, dadurch gekennzeichnet, dass man die Umsetzung in der zweiten Stufe mit 2-Amino-4,6-dimethoxypyrimidin durchführt.

3. Verfahren nach einem der Patentansprüche 1 und 2, dadurch gekennzeichnet, dass man die Umsetzung in der ersten und zweiten Stufe bei einer Temperatur von 50 bis 100 °C durchführt.

4. Verfahren nach mindestens einem der Patentansprüche 1 bis 3, dadurch gekennzeichnet, dass man in der ersten und zweiten Stufe ein inertes Lösungsmittel verwendet.

5. Verfahren nach mindestens einem der Patentansprüche 1 bis 4, dadurch gekennzeichnet, dass man die Umsetzung ohne Isolation des Zwischenproduktes der Formel III durchführt.

6. 1-Methansulfonyl-1-methyl-3-(N-methansulfonyl-N-methylaminosulfonyl)-harnstoff gemäss Formel III.

Europäisches Patentamt

# EUROPÄISCHER RECHERCHENBERICHT

Nummer der Anmeldung

EP 92 10 4612

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 131 258 (HOECHST AKTIENGESELLSCHAFT) 16. Januar 1985<br>* Beispiel 19 *<br>----- | 1 | C07D239/52<br>A01N47/36<br>C07C311/48 |
| | | | **RECHERCHIERTE SACHGEBIETE (Int. Cl.5)** |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 18 JUNI 1992 | DE JONG B.S. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)